Europäisches Patentamt

European Patent Office

Office européen des brevets

(11) Publication number: **0 127 434**
**A1**

## (12) EUROPEAN PATENT APPLICATION

(21) Application number: **84303484.4**

(22) Date of filing: **23.05.84**

(51) Int. Cl.³: **C 07 C 53/128**
**C 07 C 51/353**

(30) Priority: **27.05.83 US 499089**

(43) Date of publication of application:
**05.12.84 Bulletin 84/49**

(84) Designated Contracting States:
**BE CH DE FR GB IT LI LU NL SE**

(71) Applicant: **ELI LILLY AND COMPANY**
**307, East McCarty Street**
**Indianapolis Indiana 46285(US)**

(72) Inventor: **Harper, Richard Waltz**
**4470 Delaware Street**
**Indianapolis Indiana 46205(US)**

(72) Inventor: **Jerris, Paula Jean**
**5753 Chensford Drive, No.2B**
**Indianapolis Indiana 46226(US)**

(74) Representative: **Hudson, Christopher Mark et al,**
**Erl Wood Manor**
**Windlesham Surrey GU20 6PH(GB)**

(54) Synthesis of a branched alkanoic acid.

(57) 2-Ethyl-2-methylbutanoic acid is prepared in excellent yield by the reaction of 2-ethylbutan-1-ol with formic acid in the presence of sulfuric acid.

EP 0 127 434 A1

## SYNTHESIS OF A BRANCHED ALKANOIC ACID

This invention relates to an improved synthesis of branched alkanoic acids, more specifically 2-ethyl-2-methylbutanoic acid, a useful intermediate.

The present invention is a special embodiment of the Koch acid process for carbonylation, and is particularly favorable because of the high yields which it gives, and the high selectivity for the desired product which the process exhibits. In contrast, it has been found that the Koch process often gives complex mixtures of many products when carried out on other substrate compounds. For example, Ordyan et al., Zhur. Org. Khim. 18, 1634-37 (1982) carried out the Koch process, but using ethyl formate instead of formic acid, with 1-hexanol as the substrate. They obtained both acids and esters, and in most of their experiments they obtained major amounts of three different acids (and their esters), as well as other, unidentified products.

According to the present invention there is provided a process for preparing 2-ethyl-2-methylbutanoic acid comprising reacting 2-ethylbutan-1-ol with formic acid in the presence of sulfuric acid, and quenching the reaction mixture with water.

In this document, all temperatures are described in degrees Celsius.

The butanoic acid which is the product of the present invention is known in the art, and is best used as an intermediate, more particularly an intermediate

X-6144

-2-

in the preparation of a series of herbicides which are 5-(2,6-disubstituted-benzamido)-3-(1-ethyl-1-methylpropyl)isoxazoles.  Such herbicides are disclosed in European Patent Specification No. 81304225.6.  Example 1 of that document shows an alkyl ester of the present butanoic acid used as the starting material in the synthesis of a preferred product.

The starting compound for the present invention, 2-ethylbutan-1-ol, is now an article of commerce.

The process of the present invention is preferably carried out at a temperature in the range of from about 0° to about 25°, although temperatures as high as 50° may be used if desired.  Most preferably, it is carried out at a moderate temperature in the range of from about 10° to about 20°.  It should be noted that both the reaction with formic acid and the quenching step of the reaction are exothermic, and adequate cooling of the reaction vessel is necessary. Of course, the use of ice as part or all of the quench-water is desirable to assist in cooling the mixture.

It has been observed that the process is one in which excellent agitation is extremely important, to avoid localized excessive concentration of reactants. Strong mechanical stirring in a baffled flask is effective in bench-scale preparations; many types of industrial-scale agitated reactors are capable of providing the same intensity of agitation.

A moderate excess of formic acid is preferably used in the present process.  Excess amounts in the range of from about 10% to about 75% are quite adequate, and may be used as is convenient in a given case. It is most preferred to use excess amounts in the range of from about 25% to about 50%.

It is preferred to operate the process with concentrated sulfuric acid as the only solvent. The amount of sulfuric acid can conveniently be in the range of about 0.5-1.5 liter per mole of starting compound, to provide a fluid reaction mixture to which the necessary vigorous agitation can be imparted.

It is preferred that both the formic acid and the sulfuric acid should be highly concentrated, in the range of about 98% formic acid and about 96% sulfuric acid. These concentrations, of course, are those of commercially obtainable concentrated acids.

It has been found preferable to add the 2-ethylbutan-1-ol to the sulfuric acid as a solution in some or all of the formic acid. It is very important to add the last reactant in a small stream with excellent agitation, and to take care that the point in the reaction mixture at which the reactant is added is ome at which the agitation is most effective. The operator should also take care that agitation does not fall off as the level in the reactor rises through successive additions.

The reaction with formic acid is usually complete in a few hours time. Of course, the time of the reaction depends upon the temperature, and to a lesser extent on the amount of excess formic acid used and on the concentration of the reaction mixture. As usual, in a given case, the operator may choose to use longer reaction times to optimize the yield, or to use shorter reaction times to optimize throughput of the reactor. The choice depends on the circumstances existing at the time.

It may be desirable to use an inert organic solvent in the reaction mixture. While it is usually preferred to use only sulfuric acid as the solvent, in a given instance, such as when the available agitation is marginal, the dilution of the mixture might be desirable. The solvent in such a case must be chosen from those which are inert to the extremely severe reaction conditions. Such solvents could be found among the alkanes, such as the various isomeric hexanes, heptanes and octanes.

When the reaction with formic acid has gone as far toward completion as is desired, the mixture is quenched by pouring it into a relatively large amount of water or ice, with excellent agitation. The maintenance of a moderate to low temperature in the range of from about 0° to about 50°, preferably from about 0° to about 25° is preferred. Only brief agitation with the water is necessary.

The product is easily isolated by extraction with an alkane, such as hexane. It is desirable to clean up the product by extracting the alkane extract with aqueous base, and then acidifying, as is conventional in the synthesis of organic acids.

When the process is carried out according to the preferred methods, about 75% of the product mixture is the desired 2-ethyl-2-methylbutanoic acid, and about 25% is 2,2-dimethylpentanoic acid. The desired product can be obtained in more purified form by vacuum distillation, if desired, or the intermediate may be used as obtained from the present synthesis, and the purification carried out, as by differential crystallization, at a later point in the synthesis in which the intermediate is to be used.

The following Example illustrates a preferred process of the present invention.

## Example

A 1-liter baffled flask was charged with 270 ml. of 96% sulfuric acid at 15°, and to it was added 3 ml. of 98% formic acid, dropwise. The temperature of the mixture was held at 15-20° while a solution of 25.5 g. of 2-ethylbutan-1-ol in 46 g. of 98% formic acid was added, with vigorous agitation, over a period of 1.5 hours. The mixture was stirred for 4 hours at constant temperature after the addition, and the reaction was then quenched by pouring the mixture over 1 kg. of crushed ice with agitation. The aqueous mixture was then extracted with 500 ml. of hexane, and the aqueous layer was extracted twice with 150 ml. portions of hexane. The organic layers were combined, and extracted twice with 175 ml. portions of 1.4N potassium hydroxide containing 50 g. of crushed ice. The aqueous alkaline extracts were combined, washed with 100 ml. of hexane, and made acidic to pH 1-2 with concentrated hydrochloric acid. The acidic solution was then washed with 150 ml. and 100 ml. portions of hexane, and the organic layers were combined, washed with 75 ml. of water, dried over magnesium sulfate and evaporated under vacuum to obtain 25.6 g. of product mixture, which was analyzed by high performance capillary gas chromatography on a 10-meter methyl silicone column, using a flame ionization detector, and eluting the sample with chloroform.  The analysis showed that only two products were present, and that the mixture contained 75.65% of 2-ethyl-2-methylbutanoic acid, and 24.35% of 2,2-dimethylpentanoic acid.

## CLAIMS

1. A process for preparing 2-ethyl-2-methylbutanoic acid comprises reacting 2-ethylbutan-1-ol with formic acid in the presence of sulfuric acid, and quenching the reaction mixture with water.

2. A process according to claim 1, wherein the sulfuric acid is concentrated sulfuric acid.

3. A process according to claim 1 or 2, wherein the formic acid is concentrated formic acid.

4. A process according to any one of claims 1 to 3 wherein no organic solvent is used.

5. A process according to any one of claims 1 to 4, wherein the operating temperature is from about 0° to about 50°.

6. A process according to any one of claims 1 to 5, wherein the operating temperature is from about 0° to about 25°.

## DOCUMENTS CONSIDERED TO BE RELEVANT

EP 84303484.4

| Category | Citation of document with indication, where appropriate, of relevant passages | Relevant to claim | CLASSIFICATION OF THE APPLICATION (Int. Cl. ³) |
|---|---|---|---|
| X | CH - A - 382 133 (IMPERIAL CHEMICAL INDUSTRIES LIMITED) <br><br> * Example 1; claim; subclaims 1,4 * <br> -- | 1-6 | C 07 C 53/128 <br> C 07 C 51/353 |
| A | US - A - 3 910 963 (Y. SOUMA et al.) <br><br> * Column 6, line 57 - column 8; example 19 * <br> -- | 1,2,4-6 | |
| A | GB - A - 1 241 052 (BP CHEMICALS (U.K.) LIMITED) <br><br> * Examples * <br> ---- | 1,2,4-6 | |

| TECHNICAL FIELDS SEARCHED (Int. Cl. ³) |
|---|
| C 07 C 51/00 <br> C 07 C 53/00 |

The present search report has been drawn up for all claims

| Place of search | Date of completion of the search | Examiner |
|---|---|---|
| VIENNA | 30-08-1984 | HOFBAUER |